(19) Europäisches Patentamt
European Patent Office
Office européen des brevets

(11) **EP 4 207 213 A1**

(12) **EUROPEAN PATENT APPLICATION**

(43) Date of publication:
**05.07.2023 Bulletin 2023/27**

(21) Application number: **22214925.4**

(22) Date of filing: **20.12.2022**

(51) International Patent Classification (IPC):
**G16H 20/30** (2018.01)  **A63B 24/00** (2006.01)

(52) Cooperative Patent Classification (CPC):
**G16H 20/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2021 KR 20210190376**
**14.11.2022 KR 20220152000**

(71) Applicant: **DRAX Inc.**
**Anyang-si, Gyeonggi-do 14086 (KR)**

(72) Inventor: **YOO, Seon Kyung**
**Seoul (KR)**

(74) Representative: **Mewburn Ellis LLP**
**Aurora Building**
**Counterslip**
**Bristol BS1 6BX (GB)**

(54) **AI EXERCISE GUIDANCE DEVICE AND METHOD**

(57) An artificial intelligence (AI) exercise guidance device includes: a personal maximum weight (PMW) receiver configured to receive a $PMW_{input}$ of a user; and a PMW correction portion configured to correct the $PMW_{input}$ to a $PMW_{AI}$ based on a user objectification index, wherein the user objectification index is a numerical value calculated based on exercise data obtained during a predetermined time period.

EP 4 207 213 A1

**Description**

CROSS-REFERENCE TO RELATED APPLICATIONS

This application is based on and claims priority under 35 U.S.C. §119 to Korean Patent Application Nos. 10-2021-0190376, filed on December 28, 2021, in the Korean Intellectual Property Office, and 10-2022-0152000, filed on November 14, 2022, in the Korean Intellectual Property Office, the disclosures of which are incorporated by reference herein in their entirety.

BACKGROUND

1. Field

One or more embodiments relate to a method of automatically providing a target weight of an exercise machine by taking into account user's personal characteristics.

2. Description of the Related Art

Various types of weight training machines are provided according to parts of the body for increasing muscular strength or use purposes, and the weight training machines are mainly realized to train the upper half of the body or the lower half of the body by using hands or feet. A user may perform exercise by moving a selected weight by using an exercise structure of an exercise machine.

However, it may be difficult for a user to identify whether the user is properly using the exercise machine according to the user's physical characteristics, the user's exercise competence, or the purpose of the exercise machine.

SUMMARY

According to one or more embodiments, an artificial intelligence (AI) exercise guidance device may automatically correct a $PMW_{input}$ of maximum muscular strength information known to a user or previously measured and input by the user, based on exercise data of the user obtained during a recent predetermined time period, and thus, may provide maximum muscular strength information corrected appropriately for a user's physical strength state.

According to one or more embodiments, the AI exercise guidance device may further collect, from a user, a subjectification index including information subjectively felt by the user or data, such as injuries, pains, etc. difficult to be objectively collected, and thus, may provide maximum muscular strength information appropriate for a user's felt state of physical strength.

According to one or more embodiments, the AI exercise guidance device may automatically set a target weight of an exercise machine according to an exercise purpose of a user based on maximum muscular strength information corrected appropriately for a user's physical strength state.

According to one or more embodiments, the AI exercise guidance device may learn and analyze an objectification index for each individual user and may continually update a target weight of an exercise machine that is differently set for each user, in order to provide, to the user, a method of using the exercise machine in an optimized way.

Additional aspects will be set forth in part in the description which follows and, in part, will be apparent from the description, or may be learned by practice of the presented embodiments of the disclosure.

According to one or more embodiments, an artificial intelligence (AI) exercise guidance device includes: a personal maximum weight (PMW) receiver configured to receive a $PMW_{input}$ of a user; and a PMW correction portion configured to correct the $PMW_{input}$ to a $PMW_{AI}$ based on a user objectification index, wherein the user objectification index is a numerical value calculated based on exercise data obtained during a predetermined time period.

The AI exercise guidance device may further include a PMW guidance portion configured to re-set the $PMW_{AI}$ as a $PMW_{guidance}$ by further reflecting a user subjectification index, wherein the subjectification index includes response data of the user with respect to a question included in a questionnaire provided by a questionnaire survey portion.

The AI exercise guidance device may further include an AI exercise target setting portion configured to automatically set, based on the $PMW_{AI}$, a target weight of an exercise machine to be used by the user.

The AI exercise guidance device may further include an exercise target guidance portion configured to automatically set, based on the $PMW_{guidance}$, a target weight of an exercise machine to be used by the user.

The PMW receiver may be configured to receive a PMW manually measured by the user by using an exercise machine or a pre-stored PMW value of the user.

The user objectification index may be calculated by further taking into account an estimated value of a specific muscular strength of the user calculated by using the user's gender, age, weight, height, body mass index (BMI), and body fat percentage.

The exercise data may include at least one of a weight of an exercise machine, a number of repetitions (Reps), a number of sets, an exercise trajectory, a movement speed, and a degree of regularity of the number of repetitions (Reps) in a set unit, which are identified when the user uses the exercise machine during the predetermined time period.

When the exercise data is an exercise trajectory of an exercise machine used by the user, a degree of completion of the exercise trajectory may be determined by using an ascending start time point, a descending start time point, an ascending section average speed, a descending section average speed, and a height identified in the exercise trajectory, and the degree of completion of the exercise trajectory may be converted into a numerical value and used as the objectification index.

When the exercise data is a number of repetitions (Reps) of an exercise machine used by the user, a degree of completion of the repetitions (Reps) may be determined based on a degree of regularity between exercise trajectories of the total repetitions forming one set and a performance time in which the total repetitions forming one set are performed, and the degree of completion of the repetitions (Reps) may be converted into a numerical value and used as the objectification index.

According to one or more embodiments, an artificial intelligence (AI) exercise guidance method includes: receiving, via a personal maximum weight (PMW) receiver, a $PMW_{input}$ of a user; and correcting, via a PMW correction portion, the $PMW_{input}$ to a $PMW_{AI}$ based on a user objectification index, wherein the user objectification index is a numerical value calculated based on exercise data obtained during a predetermined time period.

BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 illustrates a smart gym system in which an artificial intelligence (AI) exercise guidance device is used, according to an embodiment;

FIG. 2 illustrates an example of an exercise machine implementing an AI exercise guidance method in a smart gym, according to an embodiment;

FIG. 3 illustrates a diagram of internal components of an exercise machine implementing an AI exercise guidance method and a smart gym server in a smart gym system, according to an embodiment;

FIG. 4 illustrates a diagram of internal components of an AI exercise guidance device, according to an embodiment;

FIG. 5 illustrates an example of a user interface of an AI exercise guidance device, according to an embodiment;

FIG. 6 illustrates an example of an exercise trajectory detected in an exercise machine implementing an AI exercise guidance method, according to an embodiment;

FIGS. 7 to 9 illustrate an example of exercise trajectories detected when first to third users of the same personal maximum weight $(PMW)_{input}$ perform twelve repetitions on a seated leg press machine during one set, according to an embodiment;

FIG. 10 illustrates an example in which a questionnaire survey portion presents a questionnaire to a user and receives response data of the user, according to an embodiment; and

FIG. 11 illustrates an example in which a PMW correction portion corrects a $PMW_{input}$ to a $PMW_{AI}$, according to an embodiment.

DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, various embodiments are described in detail with reference to the accompanying drawings, so that one of ordinary skill in the art may easily implement the disclosure.

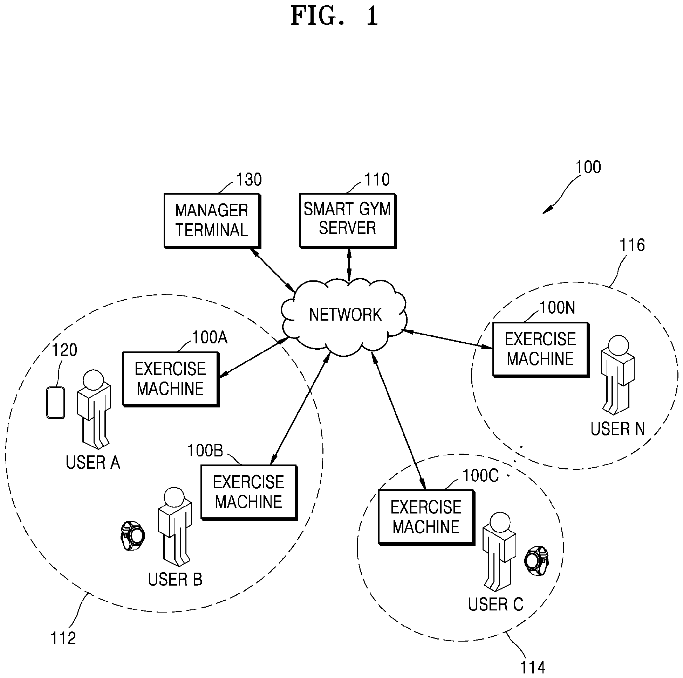

FIG. 1 illustrates a smart gym system 100 in which an artificial intelligence (AI) exercise guidance device is used, according to an embodiment.

The smart gym system 100 may include a smart gym server 110, one or more exercise machines 100a, 100b, 100c, ..., and 100n, at least one user terminal 120, and a manager terminal 130.

In FIG. 1, the smart gym server 110 may communicate with a first smart gym 112, a second smart gym 114, and an n^th smart gym 116 at different physical locations, and the exercise machines 100a and 100b arranged in the first smart gym 112 may transmit and receive data to and from the exercise machine 100c arranged in the second smart gym 114 and the exercise machine 100n arranged in the n^th smart gym 116.

According to an embodiment, a smart gym refers to a physical space in which a user may provide an exercise record using an exercise machine to the smart gym server 110, and the smart gym server 110 may learn and analyze the exercise record of the user and provide an appropriate exercise prescription to the user. The smart gym may be realized as a fitness center, a health center, a place having an exercise machine, etc.

Users (for example, USER A, USER B, USER C, ..., USER N visiting the smart gym to exercise may enter into the smart gym after an identification process. For example, the user may enter into the smart gym after verifying membership by tagging the user terminal 120 to an unmanned terminal, such as a kiosk at an entrance of the smart gym, via near-field communication (NFC) or radio frequency identification (RFID), or may enter into the smart gym after verifying membership through biometric identification such as facial recognition through the unmanned terminal, etc.

Information about the user whose membership is verified may be transmitted from the smart gym server 110 to at least one of the exercise machines 100A, 100B, 100C, ..., and 100N through a network. For example, the smart gym server 110 may transmit the information about the user to an exercise machine to which the user tags the user terminal 120. According to an embodiment, the information about the user may also be referred to by the term user data and may include at least some or all of a user's gender, age, weight, height, body mass index (BMI), and body fat percentage.

The smart gym server 110 may guide a first user USER A and a second user USER B respectively using the exercise machines 100a and 100b arranged in the smart gym 112 about an exercise method, an exercise intensity, an exercise sequence, etc. appropriate for each user. Also, the smart gym server 110 may provide values, such as a target weight, a recommended use speed, etc. of each of the exercise machines 100a and 100b. Also, the smart gym server 110 may receive exercise records of the first user USER A and the second user USER B respectively using the exercise machines 100a and 100b. Also, the smart gym server 110 may further receive, from the user terminal 120, user's health information, such as a health rate, a blood pressure, and a pulse, and user's log information.

The smart gym server 110 may be realized in the form of a cloud server. The smart gym server 110 may manage information collected from each of exercise machines in smart gym health centers at different locations in an integrated way. For example, the smart gym server 110 may manage a record of the first user using an exercise machine in the smart gym 112 at a first location and a record of the first user using an exercise machine in the smart gym 114 at a second location in an integrated way.

According to an embodiment, the one or more exercise machines 100a, 100b, 100c, ..., and 100n may be stretching exercise machines, weight training machines, or aerobic exercise machines. The weight training machines may include a free weight machine and machine equipment. The one or more exercise machines 100a, 100b, 100c, ..., and 100n may provide an appropriate exercise guide to the user through a display coupled to the exercise machines or a display capable of communicating with the exercise machines in a wired or wireless fashion. For example, in the case of a stretching machine, an exercise guide related to a stretching exercise to be performed by the user may be provided through a smart mirror capable of wired or wireless communication with the stretching machine. However, the disclosure is not limited thereto, and the exercise guide may be provided by using various output methods, such as a speaker, vibration, etc.

The one or more exercise machines 100a, 100b, 100c, ..., and 100n may perform wired or wireless communication with the smart gym server 110, the user terminal 120, and the manager terminal 130.

According to an embodiment, the user terminal 120 may be realized in the form of a smartphone, a smart watch, a hand-held device, a wearable device, etc. Also, the user terminal 120 may install an application for using the smart gym system. The user terminal 120 may receive exercise sequence information from the smart gym server 110. The exercise sequence refers to an exercise plan designed in consideration of the user's physical strength and exercise competence. The exercise sequence may include information about a list of exercise machines to be used by the user, a target weight and the number of times of uses of each exercise machine, etc.

When the user uses the one or more exercise machines 100a, 100b, 100c, ..., and 100n in the smart gym system 100, the user may perform communication by tagging the user terminal 120 via NFC or RFID or may perform an identification process by using a physical characteristic of the user. When the identification process of the user is completed, the smart gym server 110 may transmit user data to an exercise machine tagged by the user.

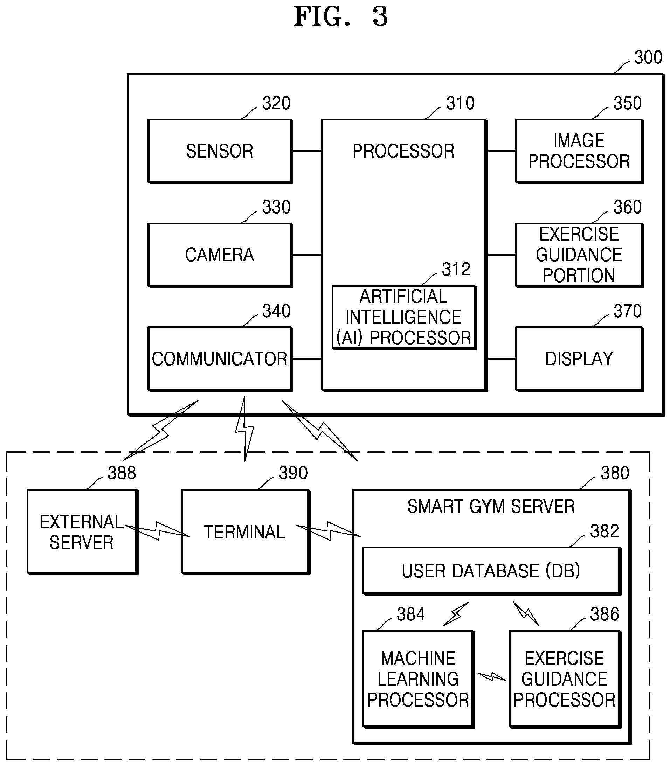

FIG. 3 illustrates a diagram of internal components of an exercise machine 300 implementing an AI exercise guidance method and a smart gym server 380 in a smart gym system, according to an embodiment.

According to an embodiment, the exercise machine 300 in a smart gym may communicate with the smart gym server 380, a user terminal 390, and an external server 388.

According to an embodiment, the exercise machine 300 may include a processor 310, a sensor 320, a communicator 340, an exercise guidance portion 360, and a display 370. Also, the exercise machine 300 may further include a camera 330 and an image processor 350. The processor 310 may further include an AI processor 312 according to necessity.

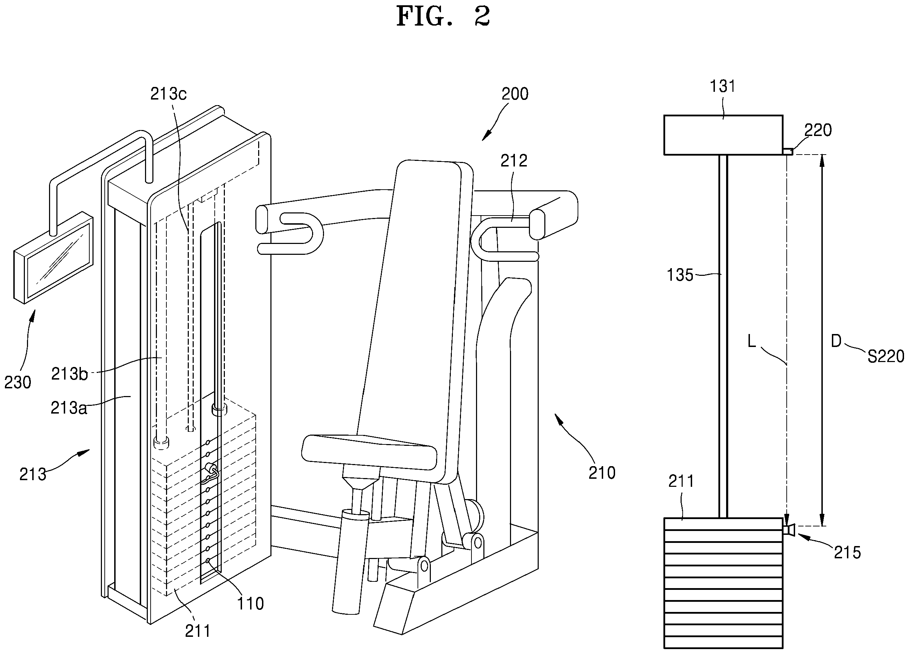

Further referring to FIG. 2, a shoulder press machine 200 indicates an example of the exercise machine 300 implementing an AI exercise guidance method used in a smart gym. The exercise machine 300 is to be described with reference to FIGS. 2 and 3.

A sensor 220 may be mounted on a frame structure 213 of an exercise body 210. The frame structure 213 may include a base frame 231a, a guide rail 231b, and a connection line 231c. The sensor 220 may irradiate a laser beam toward a pin structure 215, receive the laser beam reflected, and measure a distance D S220 from the sensor 220 to the pin structure 215 in real time or in units of a predetermined time period t. By doing so, the sensor 220 may detect, in real time, at least one of a location, a movement speed, and a moving direction of a weight member 211 selected by the fin structure 215. Also, when a user pushes a handle 212 of the shoulder press machine 200 to move a weight plate, the sensor 220 may measure the distance D S220 to the fin structure 215 stuck in the weight plate and may detect an exercise trajectory based on the measured distance.

The communicator 340 may receive a user input through a display 230 or may transmit and receive user data to and from a user database (DB) 382 of the smart gym server 380. Also, the communicator 340 may communicate with the external server 388.

The exercise guidance portion 360 may present a use reference to a user when the user uses an exercise machine. The use reference may include a movement speed guide indicating a movement speed by which an exercise machine is moved, a respiration guide indicating a respiration method when moving an exercise machine, an exercise sequence indicating a use order of exercise machines selected according to physical strength of a user, a target weight according to an exercise purpose of a user, etc.

The exercise guidance portion 360 may provide, to the user, user data received from the smart gym server 380 and information about the target weight of the exercise machine, the movement speed guide of the exercise machine, the respiration guide when using the exercise machine, the exercise sequence, the target weight according to the exercise purpose of the user, etc.

The camera 330 may be embedded in the exercise machine 300 or may communicate with the exercise machine 300 in a wired or wireless fashion. The camera 330 may capture an exercise posture of the user. The image processor 350 may analyze the exercise posture of the user captured by the camera 330 through the processor 310. Also, the AI processor 312 may learn an image processing result of the exercise posture captured when the user exercises according to the target weight of the exercise machine and transmit a result of the learning to the smart gym server 380. Also, the AI processor 312 may process or learn a user objectification index.

The smart gym server 380 may include the user DB 382, a machine learning processor 384, and an exercise guidance processor 386. The user DB 382 may store and manage the user data. The user data may include a user's gender, age, weight, height, BMI, and body fat percentage.

The machine learning processor 384 may pre-store a personal maximum weight (PMW) percentile value of an exercise machine, obtained from a population, for each exercise machine, as shown in Tables 1 to 3. According to an embodiment, the PMW refers to the muscular strength capable of being exerted by an individual against the resistance of a weight with maximum effort. Examples of the PMW may include 1RM, 4RM, etc.

Table 1, Table 2, and Table 3 respectively indicate PMW percentile values of a chest press exercise machine, a leg extension exercise machine, and a shoulder press exercise machine. The machine learning processor 384 may generate an updated population PMW by updating a PMW percentile value obtained from an initial population in predetermined units.

[Table 1]

| PMW Percentile Value | Initial Population PMW (kg) | Updated Population PMW (kg) |
| --- | --- | --- |
| PMW90 | 102.7 | 107.8 |
| ... | | |
| PMW50 | 67.2 | 67.8 |
| PMW40 | 65.1 | 64.7 |
| ... | | |
| PMW10 | 24.6 | 23.9 |

[Table 2]

| PMW Percentile Value | Initial Population PMW (kg) | Updated Population PMW (kg) |
| --- | --- | --- |
| PMW90 | 103.3 | 107.8 |
| ... | ... | ... |
| PMW50 | 67.3 | 69.2 |
| PMW40 | 66.5 | 66.8 |
| ... | ... | ... |
| PMW10 | 24.6 | 24.9 |

[Table 3]

| PMW Percentile Value | Initial Population PMW (kg) | Updated Population PMW (kg) |
| --- | --- | --- |
| PMW90 | 64.5 | 65.5 |
| ... | ... | ... |
| PMW50 | 43.3 | 43.2 |
| PMW40 | 41.2 | 40.2 |
| ... | ... | ... |
| PMW10 | 16.1 | 16.3 |

Also, the machine learning processor 384 may learn and process an objectification index including exercise records of the exercise machines used by the user in the smart gym. An example of the objectification index may include an exercise record, which may include a weight of the exercise machine, the number of repetitions (Reps), the number of sets, an exercise trajectory, a movement speed, the regularity of the number of repetitions (Reps) in a set unit, etc. identified when the exercise machine is used. Also, the objectification index may be learned and processed by selecting and adding, according to a predetermined reference, a plurality of pieces of data or indicators from the data including the weight of the exercise machine, the number of repetitions (Reps), the number of sets, the exercise trajectory, the movement speed, the regularity of the number of repetitions (Reps) in a set unit, etc.

Also, the user objectification index may further include an estimated muscular strength value, which is a user's estimated specific muscular strength calculated based on Equation 1 by using a user's gender, age, weight, height, BMI, and body fat percentage.

[Equation 1]

$$\text{ESTIMATED MUSCULAR STRENGTH VALUE} = A*\text{GENDER}+B*\text{AGE}+C*\text{BODY FAT PERCENTRAGE}+D*\text{BMI}+E$$

In Equation 1, A, B, C, D, and E indicate predetermined values.

According to an embodiment, "data" denotes raw data measured or directly input by a device. An "indicator" indicates information obtained by processing or measuring data. Also, an "index" denotes a total score generated by selecting and adding a plurality of pieces of data or indicators according to a predetermined reference.

According to another embodiment, when the machine learning processor 384 uses the number of repetitions (Reps) as the objectification index, the machine learning processor 384 may determine a degree of completion of the repetitions (Reps) based on the regularity between exercise trajectories of the total repetitions forming one set, and may use the degree of completion of the repetitions (Reps) converted into a numerical value.

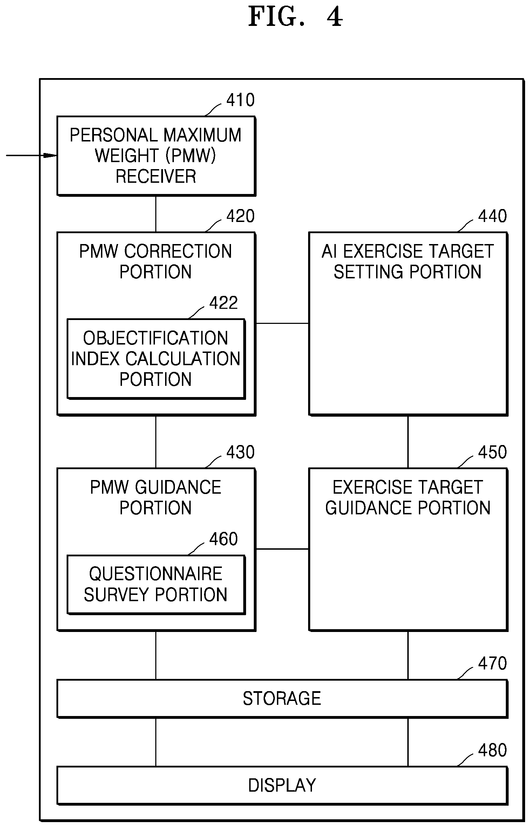

FIG. 4 illustrates a diagram of internal components of an AI exercise guidance device 400, according to an embodiment.

The AI exercise guidance device 400 may automatically correct a $\text{PMW}_{input}$ received from a user to a $\text{PMW}_{AI}$, based on an objectification index of the user obtained during a predetermined time period. Also, the AI exercise guidance device 400 may further correct the $\text{PMW}_{AI}$ to a $\text{PMW}_{guidance}$ by further reflecting a subjectification index of the user based on response data of the user with respect to a questionnaire after exercise.

According to an embodiment, the AI exercise guidance device 400 may calculate exercise data of the user obtained during a predetermined time period as the objectification index through deep learning or machine learning and may correct the $PMW_{input}$ received from the user to the $PMW_{AI}$ by automatically reflecting the calculated objectification index. In other words, the AI exercise guidance device 400 may automatically adjust a $PMW_{input}$ of a previous maximum muscular strength of the user provided by the user or pre-stored as a $PMW_{AI}$ of a recent maximum muscular strength in compliance with the physical strength of the user. Also, the AI exercise guidance device 400 may present an exercise machine appropriate for the user by further reflecting a subjectification index corresponding to feedback of the user with respect to fatigue, a limit, an injury, a pain, etc. of the user and may automatically set a target weight of the presented exercise machine.

The AI exercise guidance device 400 may include a PMW receiver 410, a PMW correction portion 420, a PMW guidance portion 430, an AI exercise target setting portion 440, and an exercise target guidance portion 450. Also, the AI exercise guidance device 400 may further include a questionnaire survey portion 460, a storage 470, and a display 480. The PMW correction portion 420 may further include an objectification index calculation portion 422. Each component may have the following function.

The PMW receiver 410 may receive a $PMW_{input}$ of a user. The $PMW_{input}$ may include a PMW manually measured by the user by using an exercise machine, a PMW of the user pre-stored in the smart gym server 380, or a PMW directly input by the user.

The PMW correction portion 420 may provide a $PMW_{AI}$ generated by correcting the $PMW_{input}$ based on a user objectification index. The user objectification index may indicate a numerical value calculated based on exercise data obtained during a predetermined time period. The $PMW_{AI}$ is a value obtained by correcting the $PMW_{input}$ based on the user objectification index and may indicate the muscular strength capable of being exerted by an individual against the resistance of a weight with maximum effort.

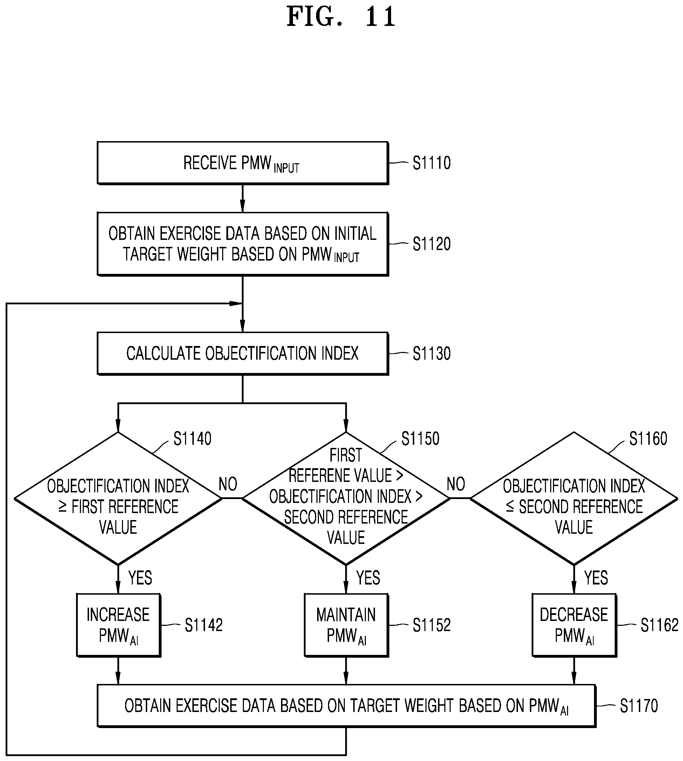

The PMW correction portion 420 may use a PMW percentile value pre-stored for each exercise machine in the machine learning processor 384, in order to correct the $PMW_{input}$. An example in which the PMW correction portion 420 corrects the $PMW_{input}$ to a $PMW_{AI}$ is described with reference to FIG. 11.

The PMW receiver 410 may receive 65 kg as a $PMW_{input}$ of a chest press exercise machine in operation S1110. In this case, the PMW correction portion 420 may detect a PMW percentile value to which 65 kg of the chest press exercise machine belongs. Referring to Table 3, the PMW correction portion 420 may detect that the $PMW_{input}$ of 65kg of the chest press exercise machine of the user corresponds to a PMW percentile value of 40.

In operation S1120, the PMW correction portion 420 may receive, from the user DB 382, the exercise data obtained during a predetermined time period when the user exercised with an initial target weight configured based on the $PMW_{input}$ of 65 kg of the chest press exercise machine. The initial target weight may be configured by reflecting an exercise purpose of the user received through user interfaces 541 to 544. According to whether the exercise purpose of the user is a free exercise 541, a standard exercise 542, muscular enhancement 543, or conditioning 544, each target weight of the exercise machine may be differently configured. For example, when the exercise purpose of the user is muscular enhancement, the initial target weight may be configured as the $PMW_{input}$ * $P_{muscular\ enhancement}$ (%). In the case of $P_{muscular\ enhancement}$ (%), the initial target weight may be configured as 65 % to 85 %, and the number of repetitions may be configured as 6 to 12 times. However, this is only an embodiment, and it may be noted that the disclosure is not limited thereto.

Also, the PMW correction portion 420 may calculate an objectification index through the objectification index calculation portion 422.

The objectification index calculation portion 422 may calculate the objectification index by using at least some of a weight of an exercise machine, the number of repetitions (Reps), the number of sets, an exercise trajectory, a movement speed, and the regularity of the repetitions (Reps) in a set unit, which are identified when the user uses the exercise machine during a predetermined time period.

When the objectification index is greater than or equal to a first reference value, the PMW correction portion 420 may correct the $PMW_{AI}$ to a value greater than the $PMW_{input}$ in operations 1140 and S1142, and when the objectification index is less than or equal to a second reference value, the PMW correction portion 420 may correct the $PMW_{AI}$ to a value less than the $PMW_{input}$ in operations S1160 and S1162. When the objectification index is less than the first reference value and greater than the second reference value, the $PMW_{AI}$ may be corrected to a value equal to the $PMW_{input}$ in operations S1150 and S1152. Also, the PMW correction portion 420 may newly update the $PMW_{AI}$ by calculating the objectification index based on exercise data obtained in operation S1170 in units of a predetermined period. In this case, when the objectification index is greater than or equal to the first reference value, the $PMW_{AI}$ may be increased, when the objectification index is less than the first reference value and greater than the second reference value, the $PMW_{AI}$ may be maintained, and when the objectification index is less than or equal to the second reference value, the $PMW_{AI}$ may be decreased.

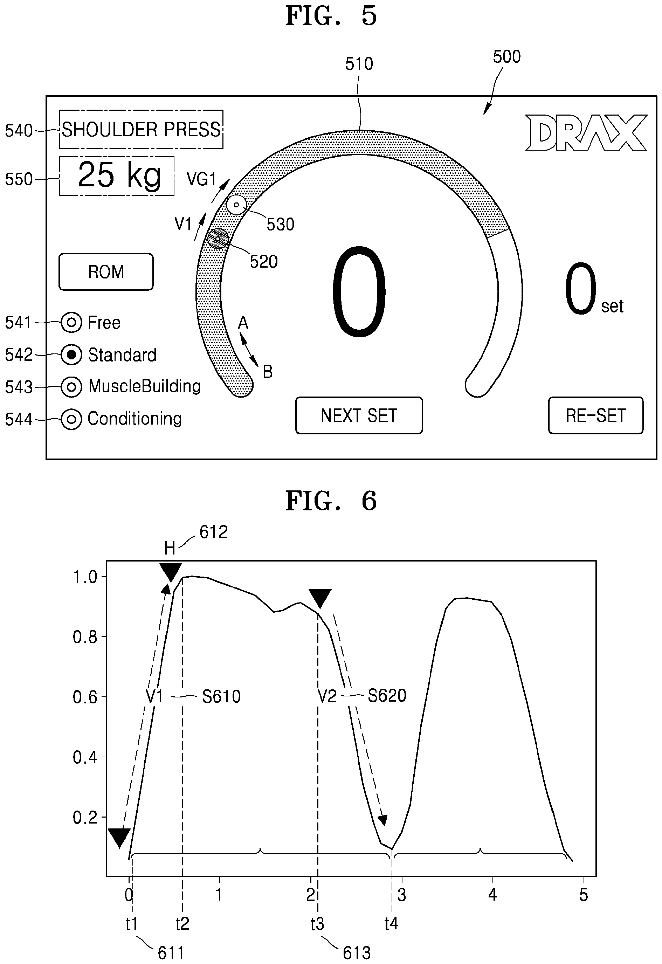

An example in which the objectification index calculation portion 422 may calculate the objectification index by using an exercise trajectory is described below. Referring to FIG. 6, the objectification index calculation portion 422 may determine a degree of completion of the exercise trajectory by using at least some of an ascending start time point 611, a descending start time point 613, an ascending section speed V1 S610, a descending section speed V2 S620, an ascending section average speed, a descending section average speed, and a height H 612, which are identified in the exercise trajectory detected while the user uses an exercise machine, and may convert the degree of completion of the exercise trajectory into the objectification index by converting the degree of completion of the exercise trajectory into a numerical value.

For example, the objectification index calculation portion 422 may determine that the ascending start time point 611 is appropriate, when the ascending start time point 611 is 0 to $t_a$ seconds according to a predetermined reference, and determine that the ascending start time point 611 is delayed, when the ascending start time point 611 deviates from $t_a$. Also, a grade of good, fair, and poor or a score in the range of 1 point to 10 points may be assigned to with respect to the degree of completion, according to a predetermined reference appropriate for the ascending start time point 611. Likewise, the ascending section speed V1 S610 may be determined to be appropriate, fast, and slow, respectively, when the ascending section speed V1 S610 is between Va and Vb, exceeds Vb, and falls short of Va, according to a predetermined reference, and the grade of good, fair, and poor or the score in the range of 1 point to 10 points may be assigned to each determination result of appropriate, fast, or slow.

Based on this method, the objectification index calculation portion 422 may determine the degree of completion of the exercise trajectory detected when the user uses the exercise machine, by using at least some of the ascending start time point 611, the descending start time point 613, the ascending section speed V1 S610, the descending section speed V2 S620, the ascending section average speed, the descending section average speed, and the height H 612.

For example, when the objectification index calculated based on the exercise trajectory with respect to the initial target weight value of the user of the $PMW_{input}$ of 65 kg of the chest press corresponds to a score between 8 points and 10 points out of a maximum of 10 points, the PMW correction portion 420 may update the $PMW_{AI}$ as a value greater than the $PMW_{input}$. For example, the $PMW_{AI}$ may be updated as a one-step higher percentile rank from the percentile value to which the $PMW_{input}$ belongs. When the percentile value to which the $PMW_{input}$ of 65 kg belongs is PMW40, the PMW correction portion 420 may update the $PMW_{AI}$ as 67.2 kg corresponding to PMW50. However, it may be noted that the percentile value may be variously designed as 1 unit, 5 units, 10 units, etc.

The PMW correction portion 420 may confirm the $PMW_{AI}$ as the $PMW_{input}$, when the objectification index calculated based on the exercise trajectory with respect to the initial target weight value of the user of the $PMW_{input}$ of 65 kg of the chest press corresponds to a score between 5 points to 7 points.

When the objectification index calculated based on the exercise trajectory with respect to the initial target weight value of the user of the $PMW_{input}$ of 65 kg of the chest press corresponds to a score between 1 point to 4 points, the PMW correction portion 420 may update the $PMW_{AI}$ as a one-step lower percentile rank from the percentile value to which the $PMW_{input}$ belongs. When the percentile value to which the $PMW_{input}$ of 65 kg belongs is PMW40, the PMW correction portion 420 may update the $PMW_{AI}$ as a weight corresponding to PMW30.

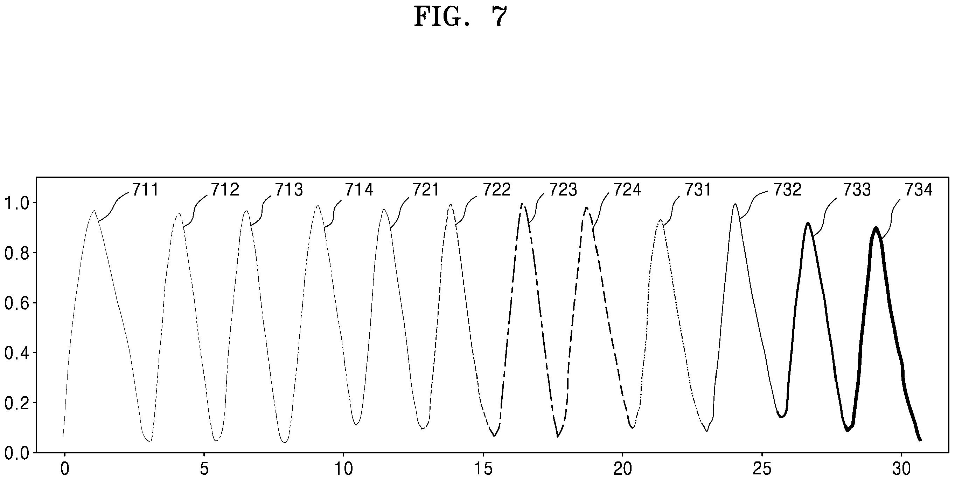

According to another embodiment, FIGS. 7 to 9 illustrate an example in which the PMW correction portion 420 uses the repetitions (Reps) as the objectification index.

The objectification index calculation portion 422 may determine the degree of completion of the repetitions (Reps) based on the regularity between exercise trajectories of the total number of repetitions forming one set and may calculate the objectification index by converting the degree of completion of the repetitions (Reps) into a numerical value. Also, the objectification index calculation portion 422 may calculate the objectification index by further referring to a performance time in which the total repetitions forming one set are performed.

FIGS. 7 to 9 illustrate an example of an exercise trajectory detected when each of first to third users of the same $PMW_{input}$ perform twelve repetitions on a seated leg press during one set. In FIGS. 7 to 9, an x axis indicates a time, and a y axis indicates a movement displacement of the exercise machine.

The objectification index calculation portion 422 may determine whether all of the twelve repetitions in one set are performed or only part is performed with a user giving up the exercise. Also, the objectification index calculation portion 422 may determine a degree of completion of the repetitions (Reps), based on the regularity between exercise trajectories of each of the twelve repetitions 711 to 714, 721 to 724, and 731 to 734 of the first user, the twelve repetitions 811 to 814, 821 to 824, and 831 to 834 of the second user, and the twelve repetitions 911 to 914, 921 to 924, and 931 to 934 of the third user. In this case, the determination may be performed based on the division of early stage regularity, middle stage regularity, and latter stage regularity.

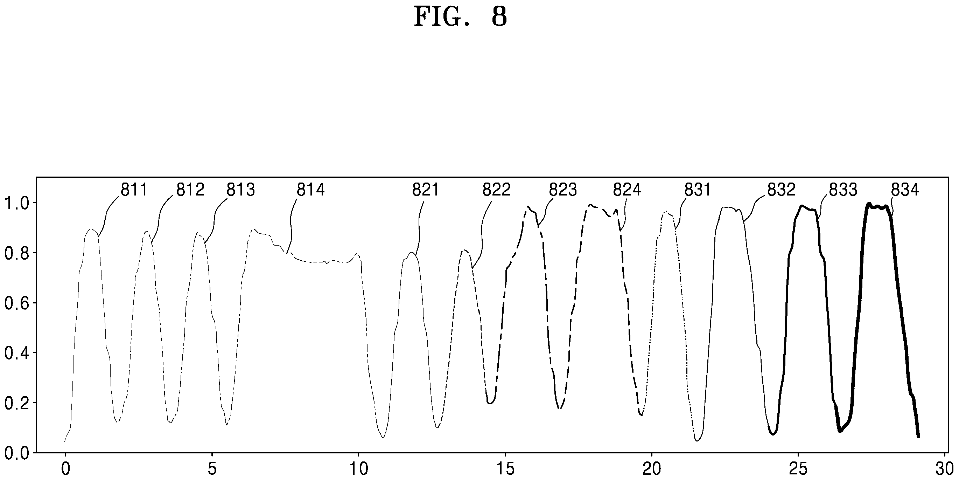

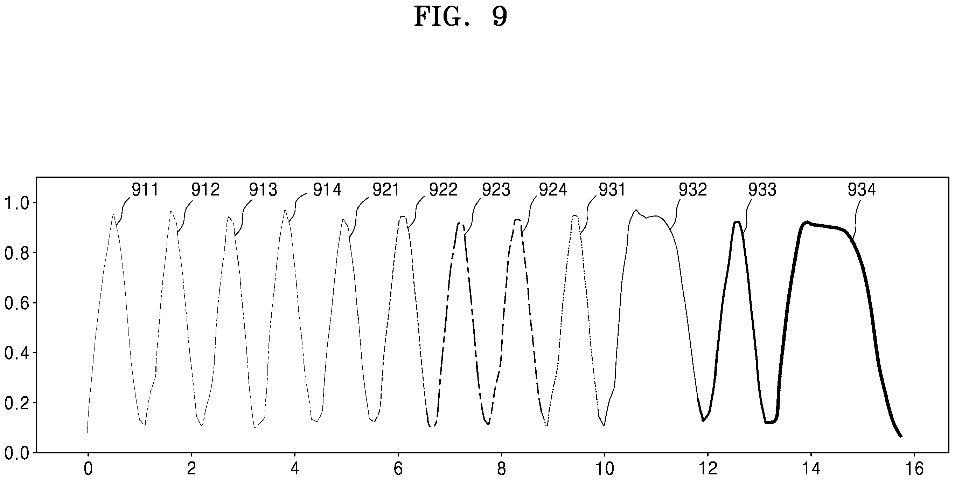

For example, the early stage regularity of the first user of FIG. 7 and the third user of FIG. 9 with respect to the first to fourth times 711 to 744 and 911 to 914 show a relatively high value, with the four exercise trajectories are substantially constant. In this case, four points out of a maximum of four points may be assigned to the early stage regularity. The second user of FIG. 8 shows constant exercise trajectories with respect to the first to third times 811 to 813, but a highly deviating exercise trajectory with respect to the fourth times 814, and thus, it is difficult to deem the early stage regularity as high. In this case, three points out of a maximum of four points may be assigned to the early stage regularity. However, this is only an embodiment in which the early stage regularity is determined and a numerical value is assigned thereto by determining whether or not the exercise trajectories are constant. Various methods, such as a distance between exercise trajectories, a length on a time series, etc., may be used to determine the regularity

The objectification index calculation portion 422 may determine that the first user has a high degree of regularity with respect to the twelve repetitions, the second user has a low middle stage regularity, with respect to the fourth to seventh repetitions, and the third user has a low regularity, with respect to the tenth to twelfth repetitions, that is, a low latter stage regularity, and may assign scores corresponding thereto.

The objectification index calculation portion 422 may determine each of a standard deviation of an ascending start time point, a standard deviation of a descending start time point, a high standard deviation, a standard deviation of an ascending section speed, a standard deviation of a descending section speed, etc. in each of the exercise trajectories of the twelve repetitions of each user and may assign a score according to a predetermined reference.

The objectification index calculation portion 422 may calculate the objectification index by further referring to a performance time in which the total repetitions forming one set are performed.

Based on the objectification index calculated by the objectification index calculation portion 422, the PMW correction portion 420 may set a different $PMW_{AI}$ for each of the first to third users, by reflecting a recent state of physical strength of each of the first to third users of the same $PMW_{input}$.

The PMW correction portion 420 may apply various methods, such as statistical processing for using, as the objectification index, a plurality of pieces of data from among variables, such as a weight of an exercise machine, the number of repetitions (Reps), the number of sets, an exercise trajectory, a movement speed, and a degree of regularity of the repetitions (Reps) in a set unit, or addition of the variables by assigning a weight to each variable according to a predetermined rule.

The PMW guidance portion 430 may re-set a $PMW_{guidance}$ by further reflecting a user subjectification index in the $PMW_{AI}$. The PMW guidance portion 430 may be activated according to selection of a user. When the user responds to a questionnaire by activating the questionnaire survey portion 460, the exercise target guidance portion 450 may re-set, based on response data of the user, the $PMW_{guidance}$ for each exercise machine.

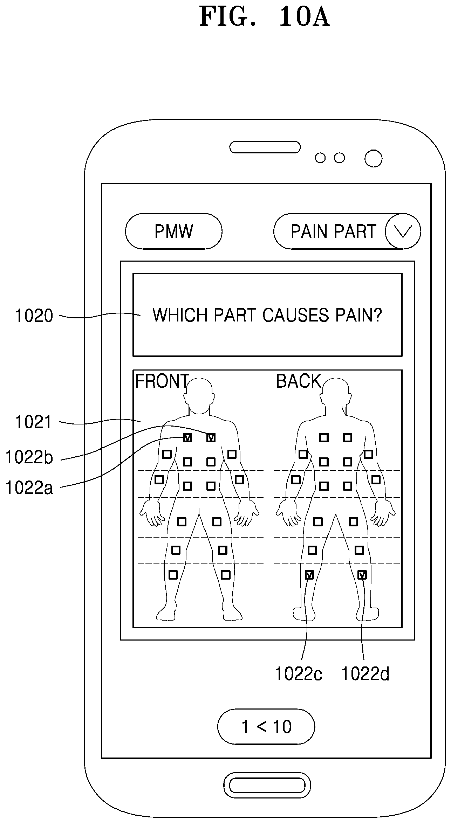

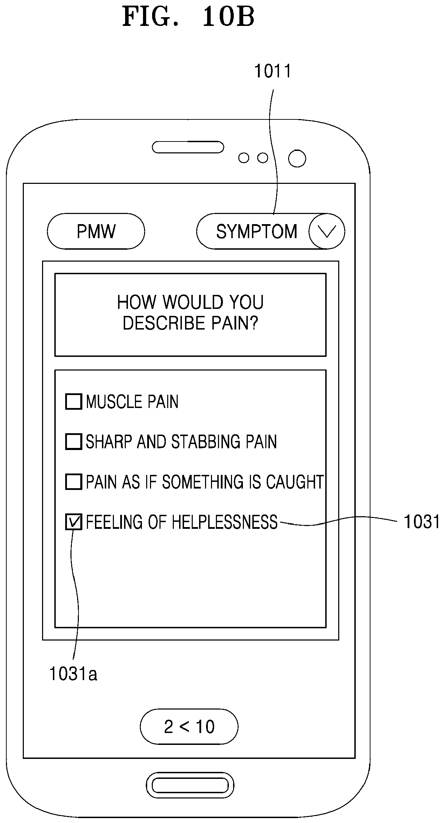

The subjectification index may include the response data of the user with respect to questions included in the questionnaire provided by the questionnaire survey portion 460. FIG. 10 illustrates an example in which a questionnaire survey portion 1000 presents a questionnaire to a user and receives response data 1021 and 1031 of the user, according to an embodiment.

The questionnaire survey portion 1000 may include question portions 1020 and 1030 presenting the questionnaire to the user and response portions 1021 and 1031 receiving response data 1022a, 1022b, 1022c, 1022d, and 1031a of the user.

The PMW guidance portion 430 may re-set a $PMW_{guidance}$ by further reflecting a user subjectification index in a $PMW_{AI}$. The subjectification index may include the response data 1022a, 1022b, 1022c, 1022d, and 1031a of the user with respect to questions 1020 and 1030 included in the questionnaire provided by the questionnaire survey portion 1000.

For example, when, after presenting a question about a "pain part" to a user, the response data 1022a, 1022b, 1022c, and 1022d with respect to the pain part is received from the user, the PMW guidance portion 430 may re-set the $PMW_{AI}$ of an exercise machine using muscles corresponding to the pain part as the $PMW_{guidance}$.

In detail, the storage 470 may pre-store at least one muscle type activated when an exercise machine is used, a degree of contribution of the at least one muscle type, and an exercise trajectory guide generated when an exercise machine is used appropriately according to a reference of use. Also, the storage 470 may store information generated by the PMW receiver 410, the PMW correction portion 420, the PMW guidance portion 430, the AI exercise target setting portion 440, or the exercise target guidance portion 450. The PMW guidance portion 430 may re-set the $PMW_{AI}$ of the exercise machine using the muscle type corresponding to the pain part as the $PMW_{guidance}$ by using the information pre-stored in the storage 470.

Referring to FIG. 10, when the user has pains in upper chests 1022a and 1022b and gastrocnemius 1022c and 1022d, and a pain level of the upper chests 1022a and 1022b corresponds to 5 points out of a maximum of 10 points (the maximum pain), the PMW guidance portion 430 may re-set the $PMW_{AI}$ of the exercise machine activating the upper chests 1022a and 1022b as the $PMW_{guidance}$ according to a predetermined criterion. The PMW guidance portion 430 may identify a list of exercise machines activating the upper chests through the storage 470. The PMW guidance portion 430 may re-set the $PMW_{AI}$ of exercise machines of the identified list of exercise machines as the $PMW_{guidance}$. For example, when the user sends feedback as the pain level of the upper chests 1022a and 1022b being 7 points, the $PMW_{guidance}$ of the user in which the $PMW_{AI}$ of the chest press corresponds to a percentage of PMW50 may be re-set as PMW30 according to a predetermined criterion.

According to another embodiment, when it is estimated that an injury occurred with the pain level of the upper chests 1022a and 1022b being 8 points or higher, the $PMW_{guidance}$ may be re-set as 0 kg, according to a predetermined criterion, so that an exercise on the exercise machine in connection with the upper chests 1022a and 1022b may not be performed.

Based on the $PMW_{AI}$ estimated by the PMW correction portion 420, the AI exercise target setting portion 440 may automatically set a target weight of an exercise machine to be used by the user.

Based on the $PMW_{guidance}$ estimated by the PMW guidance portion 430, the exercise target guidance portion 450 may automatically set the target weight of the exercise machine to be used by the user.

The display 480 may display muscles activated when an exercise machine selected by a user is used, a target weight 550 (FIG. 5) of the exercise machine set based on the $PMW_{AI}$ or the $PMW_{guidance}$ of the AI exercise target setting portion 440 or the exercise target guidance portion 450, etc.

According to an embodiment, the AI exercise guidance device may automatically correct maximum muscular strength information known to a user or measured and input by the user, based on exercise data of the user obtained during a recent predetermined time period, and thus, may provide maximum muscular strength information corrected appropriately for a physical strength state of the user. Thus, an exercise program most appropriate for a current state of the user may be provided.

According to an embodiment, the AI exercise guidance device may further collect, from a user, a subjectification index including information subjectively felt by the user or data, such as injuries, pains, etc. difficult to be objectively collected, and thus, may provide maximum muscular strength information appropriate for a user's felt state of physical strength. Thus, injuries, etc. may be prevented.

According to an embodiment, the AI exercise guidance device may automatically set and provide a target weight of an exercise machine according to an exercise purpose of a user based on maximum muscular strength information corrected appropriately for a user's physical strength state.

According to an embodiment, the AI exercise guidance device may learn and analyze an objectification index or a subjectification index for each individual user and may continually update a target weight of an exercise machine that is differently set for each user. Thus, a method of using the exercise machine in an optimized way may be provided to the user.

The methods according to an embodiment may be implemented in the form of a program command executable by various computer devices and may be recorded on a computer-readable medium. The computer-readable medium may include a program command, a data file, a data structure, etc. individually or in a combined fashion. The program command recorded on the medium may be specially designed and configured for an embodiment or may be well known to and usable by one of ordinary skill in the art.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by one of ordinary skill in the art that various changes in form and details may be made therein without departing from the spirit and scope as defined by the following claims.

**Claims**

1. An artificial intelligence (AI) exercise guidance device comprising:

   a personal maximum weight (PMW) receiver configured to receive a $PMW_{input}$ of a user; and
   a PMW correction portion configured to correct the $PMW_{input}$ to a $PMW_{AI}$ based on a user objectification index, wherein the user objectification index is a numerical value calculated based on exercise data obtained during a predetermined time period, and PMW indicates a muscular strength capable of being exerted by an individual against the resistance of a weight with maximum effort.

2. The AI exercise guidance device of claim 1, further comprising a PMW guidance portion configured to re-set the $PMW_{AI}$ as a $PMW_{guidance}$ by further reflecting a user subjectification index,
   wherein the subjectification index includes response data of the user with respect to a question included in a questionnaire provided by a questionnaire survey portion.

3. The AI exercise guidance device of claim 1, further comprising an AI exercise target setting portion configured to automatically set, based on the $PMW_{AI}$, a target weight of an exercise machine to be used by the user.

4. The AI exercise guidance device of claim 2, further comprising an exercise target guidance portion configured to automatically set, based on the $PMW_{guidance}$, a target weight of an exercise machine to be used by the user.

5. The AI exercise guidance device of claim 1, wherein the PMW receiver is configured to receive a PMW manually measured by the user by using an exercise machine or a pre-stored PMW value of the user.

6. The AI exercise guidance device of claim 1, wherein the user objectification index is calculated by further taking into account an estimated value of a specific muscular strength of the user calculated by using the user's gender, age, weight, height, body mass index (BMI), and body fat percentage.

7. The AI exercise guidance device of claim 1, wherein the exercise data includes at least one of a weight of an exercise machine, a number of repetitions (Reps), a number of sets, an exercise trajectory, a movement speed, and a degree of regularity of the number of repetitions (Reps) in a set unit, which are identified when the user uses the exercise machine during the predetermined time period.

8. The AI exercise guidance device of claim 1, wherein, when the exercise data is an exercise trajectory of an exercise machine used by the user, a degree of completion of the exercise trajectory is determined by using an ascending start time point, a descending start time point, an ascending section average speed, a descending section average speed, and a height identified in the exercise trajectory, and the degree of completion of the exercise trajectory is converted into a numerical value and used as the objectification index.

9. The AI exercise guidance device of claim 1, wherein, when the exercise data is a number of repetitions (Reps) of an exercise machine used by the user, a degree of completion of the repetitions (Reps) is determined based on a degree of regularity between exercise trajectories of the total repetitions forming one set and a performance time in which the total repetitions forming one set are performed, and the degree of completion of the repetitions (Reps) is converted into a numerical value and used as the objectification index.

10. An artificial intelligence (AI) exercise guidance method comprising:

   receiving, via a personal maximum weight (PMW) receiver, a $PMW_{input}$ of a user; and
   correcting, via a PMW correction portion, the $PMW_{input}$ to a $PMW_{AI}$ based on a user objectification index,
   wherein the user objectification index is a numerical value calculated based on exercise data obtained during a predetermined time period.

11. The AI exercise guidance method of claim 10, further comprising re-setting, via a PMW guidance portion, the $PMW_{AI}$ as a $PMW_{guidance}$ by further reflecting a user subjectification index,
   wherein the subjectification index includes response data of the user with respect to a question included in a questionnaire provided by a questionnaire survey portion.

12. The AI exercise guidance method of claim 10, further comprising automatically setting, via an AI exercise target setting portion, based on the $PMW_{AI}$, a target weight of an exercise machine to be used by the user.

13. The AI exercise guidance method of claim 11, further comprising automatically setting, via an exercise target guidance portion, based on the $PMW_{guidance}$, a target weight of an exercise machine to be used by the user.

14. The AI exercise guidance method of claim 10, wherein the exercise data includes at least one of a weight of an exercise machine, a number of repetitions (Reps), a number of sets, an exercise trajectory, a movement speed, and a degree of regularity of the number of repetitions (Reps) in a set unit, which are identified when the user uses the exercise machine during the predetermined time period.

15. A computer-readable recording medium having stored thereon commands for a computing device to perform operations comprising:

   receiving, via a personal maximum weight (PMW) receiver, a $PMW_{input}$ of a user; and
   correcting, via a PMW correction portion, the $PMW_{input}$ to a $PMW_{AI}$ based on a user objectification index,
   wherein the user objectification index is a numerical value calculated based on exercise data obtained during a predetermined time period.

# FIG. 1

# FIG. 2

EP 4 207 213 A1

# FIG. 3

300

320
**SENSOR**

310
**PROCESSOR**

350
**IMAGE PROCESSOR**

330
**CAMERA**

360
**EXERCISE GUIDANCE PORTION**

312
**ARTIFICIAL INTELLIGENCE (AI) PROCESSOR**

340
**COMMUNICATOR**

370
**DISPLAY**

388
**EXTERNAL SERVER**

390
**TERMINAL**

380
**SMART GYM SERVER**

382
**USER DATABASE (DB)**

384
**MACHINE LEARNING PROCESSOR**

386
**EXERCISE GUIDANCE PROCESSOR**

# FIG. 4

## FIG. 5

SHOULDER PRESS

25 kg

ROM

Free

Standard

MuscleBuilding

Conditioning

NEXT SET

RE-SET

## FIG. 6

# FIG. 7

# FIG. 8

FIG. 9

EP 4 207 213 A1

# FIG. 10A

# FIG. 10B

1011

PMW

SYMPTOM ⌄

HOW WOULD YOU
DESCRIBE PAIN?

☐ MUSCLE PAIN

☐ SHARP AND STABBING PAIN

☐ PAIN AS IF SOMETHING IS CAUGHT

☑ FEELING OF HELPLESSNESS————1031

1031a

2 < 10

# FIG. 11

RECEIVE $PMW_{INPUT}$ — S1110

OBTAIN EXERCISE DATA BASED ON INITIAL TARGET WEIGHT BASED ON $PMW_{INPUT}$ — S1120

CALCULATE OBJECTIFICATION INDEX — S1130

S1140
OBJECTIFICATION INDEX ≥ FIRST REFERENCE VALUE

NO

S1150
FIRST REFERENE VALUE > OBJECTIFICATION INDEX > SECOND REFERENCE VALUE

NO

S1160
OBJECTIFICATION INDEX ≤ SECOND REFERENCE VALUE

YES → INCREASE $PMW_{AI}$ — S1142

YES → MAINTAIN $PMW_{AI}$ — S1152

YES → DECREASE $PMW_{AI}$ — S1162

OBTAIN EXERCISE DATA BASED ON TARGET WEIGHT BASED ON $PMW_{AI}$ — S1170

Europäisches
Patentamt

European
Patent Office

Office européen
des brevets

# EUROPEAN SEARCH REPORT

Application Number

**EP 22 21 4925**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/001181 A1 (VON PRELLWITZ LEONARDO [US] ET AL) 4 January 2018 (2018-01-04) * paragraphs [0002], [0004], [0032], [0038], [0047] – [0058], [0064], [0065], [0073], [0082], [0086], [0088] – [0090], [0094]; figures 1,2,4,7,10 * | 1-15 | INV. G16H20/30 A63B24/00 |
| X | US 2011/281249 A1 (GAMMELL NICHOLAS [US] ET AL) 17 November 2011 (2011-11-17) | 1-7, 10-15 | |
| A | * paragraphs [0019], [0026], [0028], [0030], [0035], [0036], [0038], [0047], [0120], [0121], [0139], [0150] – [0160] * | 8,9 | |
| A | KR 2003 0068790 A (KIM JONG GWAN [KR]) 25 August 2003 (2003-08-25) * the whole document * | 1-15 | |
| A | US 2020/391080 A1 (POWERS PHILIP [US] ET AL) 17 December 2020 (2020-12-17) * the whole document * | 1-15 | |

TECHNICAL FIELDS
SEARCHED (IPC)

G16H
A63B

The present search report has been drawn up for all claims

| Place of search | Date of completion of the search | Examiner |
|---|---|---|
| Munich | 21 April 2023 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 22 21 4925**

**21-04-2023**

| Patent document cited in search report | | Publication date | Patent family member(s) | | Publication date |
|---|---|---|---|---|---|
| US 2018001181 | A1 | 04-01-2018 | US 2018001181 | A1 | 04-01-2018 |
| | | | US 2022249937 | A1 | 11-08-2022 |
| US 2011281249 | A1 | 17-11-2011 | US 2011281249 | A1 | 17-11-2011 |
| | | | WO 2011143670 | A1 | 17-11-2011 |
| KR 20030068790 | A | 25-08-2003 | NONE | | |
| US 2020391080 | A1 | 17-12-2020 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210190376
- KR 1020220152000